Europäisches Patentamt

European Patent Office

Office européen des brevets

(11) Numéro de publication: **0 182 732**
**A2**

# (12) DEMANDE DE BREVET EUROPEEN

(21) Numéro de dépôt: 85450026.1

(22) Date de dépôt: 08.11.85

(51) Int. Cl.⁴: **G 01 N 25/14,** G 01 N 33/14

(30) Priorité: **13.11.84 FR 8417403**

(71) Demandeur: **Chenard, Pierre, 78 rue Albert-Pitres, F-33000 Bordeaux (FR)**

(43) Date de publication de la demande: **28.05.86 Bulletin 86/22**

(84) Etats contractants désignés: **AT BE CH DE GB IT LI**

(72) Inventeur: **Chenard, Pierre, 78 rue Albert-Pitres, F-33000 Bordeaux (FR)**

(54) Appareil permettant l'extraction de l'alcool et des acides volatils dans les mélanges hydro-alcooliques.

(57) Appareil permettant l'extraction de l'alcool et des acides volatils qui comporte un générateur de vapeur équipé d'une électrovanne, un barboteur surmonté d'une colonne rectificatrice et un réfrigérant.

Cet appareil est utilisé en œnologie ainsi que dans les industries alimentaires et chimiques.

EP 0 182 732 A2

La présente invention concerne un appareil permettant l'extraction de l'alcool et des acides volatiles dans les mélanges hydro-alcooliques.

Les différentes méthodes d'analyse fiable dans ce domaineconsistent à entraîner ces substances par la vapeur d'eau et les condenser dans un récipient. Si le principe est bien connu, sa réalisation représente encore pour les opérateurs de nombreuses difficultés de toutes sortes : lenteur, imprécision, erreurs multiples.

Les différents appareils connus et utilisés actuellement, notamment pour l'analyse des vins, ne permettent des analyses exactes ou sont encombrants et coûteux. Les brevets numéros 75.04.190, 77.02.304, 78.22.554, 80.27.474, notamment ne concernent que des installations industrielles de distillation. Le brevet 72.18.746 traite d'un appareil élaboré à l'effet du seul dosage de l'alcool par voie chimique.

Par ailleurs l'appareil Rettirer n'est pas conforme aux exigences analytiques actuelles, etl'appareil Jaulnes est un appareil précisément très encombrant et très coûteux.

Nous avons maintenant réalisé un appareil, simple, pratique, peu coûteux d'un entretien et d'un montage faciles qui permettra tous les dosages d'alcool et la titration des acides volatiles. D'une façon générale, il permet les séparations indispensables pour se conformer aux prescriptions analytiques européennes et internationales dans les meilleures conditions de rapidité et d'économie. En outre, il n'utilise qu'une seule source d'énergie : l'électricité.

La présente invention a donc pour objet un appareil destiné à séparer automatiquement les substances volatiles d'un mélange hydro-alcoolique par entraînement à la vapeur et muni de dispositifs permettant un fonctionnement automatique .

La description de l'appareil permettra de mieux comprendre l'invention.

Les dessins représentent à titre d'exemple non limitatif une forme d'exécution de l'objet de l'invention.

Il comprend :

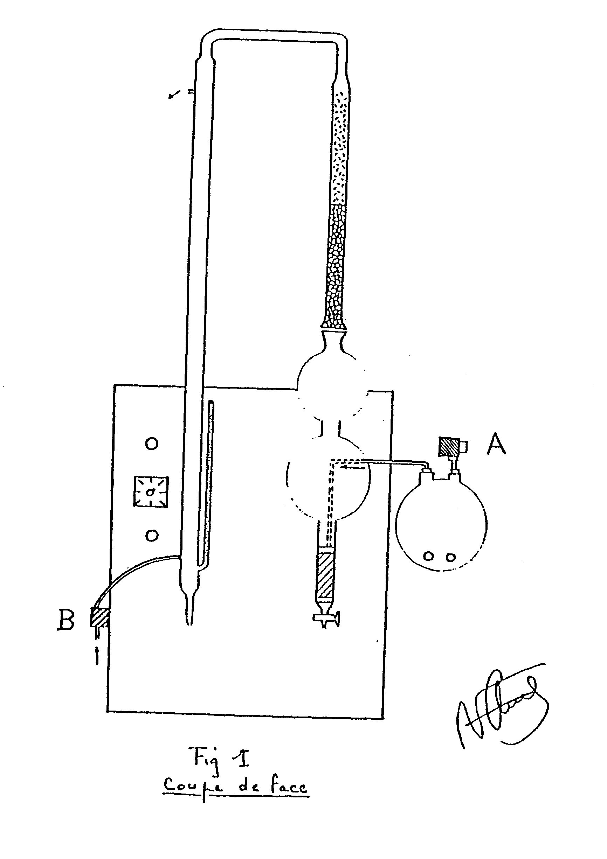

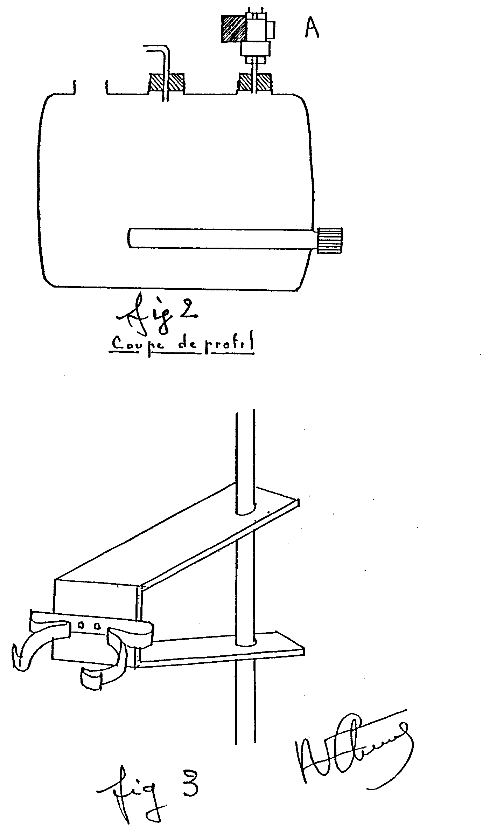

- UN GENERATEUR DE VAPEUR constitué par un cylindre en verre de diamètre 13 cm et de longueur 16 cm, il est montré en coupe de face dans la figure I et de profil dans la figure 2.

Le chauffage est assuré par deux cartouches de 1.000 Watts (alimentation en 220 volts).

Le générateur est surmonté par au moins 3 canalisations de diamètre 22

- l'une permettant le dégagement de la vapeur d'eau par une canalisation en verre de diamètre 1 cm

- l'autre est prévue pour le remplissage de l'eau permutée à la fin de chaque opération.

- la dernière est surmontée d'une vanne (A) (alimentée en 24 volts).

Cette vanne est fermée lorsque l'appareil fonctionne et s'ouvre à l'arrêt.

- UN BARBOTEUR mis en communication avec le générateur par une canalisation en verre de 1 cm de diamètre qui plonge à la partie basse de ce barboteur.

Ce dispositif comprend un ballon de 1 l surmonté d'un ballon de 200 à 250 cm$^3$ qui évite que la mousse ne passe dans la colonne rectificatrice.

A la partie inférieure se trouve un tube de diamètre 22 mm terminé par un robinet avec voie de 4 mm. Ce tube est enroulé par 10 spirales d'un conducteur (2 mm de large) qui constitue une résistance auxiliaire.

L'alimentation de cette résistance auxiliaire est en 250 Watts.

- UNE COLONNE DE RECTIFICATION raccordée au barboteur par un joint rodé assujetti par une pince.

Cette colonne d'un diamètre de 26 mm à 36 cm de haut.

Elle contient un tricot d'inox de 15 cm de haut et des anneaux de Rachy sur une hauteur également de 15 cm.

- UN REFRIGERANT raccordé à la colonne de rectification par un tube de verre coudé de 16 mm de diamètre.

Le réfrigérant possède une longueur d'au moins 60 cm et se compose de 2 tubes inox respectivement d'un diamètre extérieur de 17 mm et de 10 mm.

Une colonne adjacente aboutissant à l'extrémité inférieure du réfrigérant et remplie de billes de verre facilite l'écoulement du distillat ainsi que la récupération des premières vapeurs extraites.

Le contrôle de l'arrivée d'eau dans le réfrigérant est assuré par une électro-vanne (B).

- UN COFFRET qui sert de support et comporte :

- un transformateur qui alimente en 24 volts les deux vannes (A & B) ainsi que la résistance auxiliaire en 24 volts.

- une minuterie

- un relais temporisé

- des voyants permettant de constater que les différentes résistances sont en fonctionnement.

La liaison entre supports et verrerie se fait par clips solidaires d'une pièce plastique susceptible de coulisser sur les tiges de fixation (figure 3).

La manipulation de l 'appareil nécessite le minimum d'opérations c'est-à-dire

- un appoint d'eau dans le générateur jusqu'au trait de marque
- le remplissage du barboteur avec le mélange hydro-alcoolique à analyser·

- la rotation de la minuterie jusqu'au temps prévu

    * 3 minutes pour l'acidité volatile

    * 4 minutes pour les déterminations de l'alcool

L'appareil fonctionne sans autres manipulations.

L'arret se fait automatiquement sans aucune intervention.

Avec les caractéristiques qu'il présente cet appareil peut être parfaîtement utilisé dans l'industrie agro-alimentaire et chimique puisqu'il permet, outre l'extraction de l'alcool et de l'acidité volatile, de nombreux autres dosages notamment ceux de l'azote ammoniacal et de l'acide sorbique.

- 4 -

R E V E N D I C A T I O N S

=============

1 - Appareil permettant l'extraction automatique et le dosage de l'alcool et des matières volatiles dans un mélange hydro-alcoolique comportant un générateur de vapeur chauffé par deux cartouches de 800 à 1 200 Watts.

2 - Appareil selon la revendication 1 caractérisé en ce que le générateur est surmonté d'une vanne permettant la mise à l'air.

3 - Appareil selon la revendication 1 caractérisé en ce que le bas du barboteur est équipé d'une résistance auxiliaire de 250 Watts sous la forme d'un conducteur de 2 mm de large formant 10 spirales pour assurer la constance du niveau du mélange.

4 - Appareil selon la revendication 1 caractérisé en ce qu'il comporte soit 2 colonnes de rectification en tricot d'inox de longueur de 15 cm et de diamètre de 2,5 soit d'une seule colonne de 15 cm, prolongée par 15 cm constitués par des anneaux de Rachy.

5 - Appareil selon la revendication 1 caractérisé en ce qu'il comporte un réfrigérant en matériau inoxydable.

6 - Appareil selon la revendication 1 caractérisé en ce que tous les points de fixation sont constitués du côté de l'appareil par des clips et du côté du support par des parties plastiques trouées qui coulissent sur les tiges de soutien permettant un montage, un démontage et un réglage immédiats.

7 - Appareil selon les revendications 2 à 6 pouvant être utilisé pour des dosages dans les industries agro-alimentaires et chimiques.

0182732

Fig 1
Coupe de face

0182732

A

Fig 2

Coupe de profil

fig 3